(19) Europäisches Patentamt
European Patent Office
Office européen des brevets

(11) Publication number: 0 486 854 A1

# (12) EUROPEAN PATENT APPLICATION

(21) Application number: **91118477.8**

(51) Int. Cl.$^5$: **C07K 15/00**, A61K 9/06

(22) Date of filing: **30.10.91**

(30) Priority: **20.11.90 IT 4849290**

(43) Date of publication of application:
**27.05.92 Bulletin 92/22**

(84) Designated Contracting States:
**AT BE CH DE DK ES FR GB GR IT LI LU NL SE**

(71) Applicant: **ISTITUTO FARMACO BIOLOGICO RIPARI-GERO S.p.A.**
**Via Montearioso, 11**
**I-53035 Monteriggioni (Siena)(IT)**

(72) Inventor: **Bolasco, Franco**
**Via Montearioso 11**
**I-53035 Monteriggioni (Siena)(IT)**

(74) Representative: **Minoja, Fabrizio**
**Studio Consulenza Brevettuale Via Rossini, 8**
**I-20122 Milano(IT)**

## (54) Aqueous compositions containing calcitonin for the nasal administration.

(57) Aqueous compositions for the nasal administration, containing calcitonin and cetylpyridinium chloride, have a surprisingly high bio-availability.

EP 0 486 854 A1

The present invention relates to aqueous compositions containing calcitonin, for the nasal administration, which compositions have a higher bio-availability than that of the already known ones, such as those described in UK Pat. n: 1.354.126 and in Italian Pat. N. 1.172.324.

The cited British patent relates to "nasal" aqueous compositions (said definition will be used hereinafter for shortness) containing, besides calcitonin, sodium phosphate, citric acid, sodium chloride, glycerin, sorbitol, methyl paraben and propyl paraben. On the other hand, the Italian patent claims nasal compositions containing calcitonin, benzalkonium chloride, sodium chloride and HCl to pH 3.7. According to this patent, benzalkonium chloride allows to avoid contamination by micro-organisms, as well as to improve the bio-availability of the active ingredient.

Now, it has surprisingly been found that nasal aqueous compositions containing calcitonin and cetylpyridinium chloride, adjusted to pH 4 by means of acetate buffer, besides having a very good stability to micro-organisms, also have a markedly improved bio-availability.

Preferably, the used calcitonin is salmon calcitonin, which will be present in the compositions of the invention in amounts from 100 to 5,000 I.U., preferably from about 250 to about 2,500 I.U., per ml of ready-to-use aqueous solution.

Cetylpyridinium chloride, of formula :

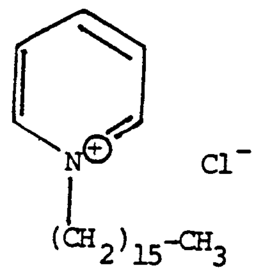

is a quaternary salt, also named Cepacol®, Cetamium®, which is in form of a water-soluble white powder melting at 77-83 °C. According to the invention, it is used in amounts ranging from 0.01 to 0.4 mg/ml of ready-to-use aqueous solution, preferably from 0.05 to 0.2 mg/ml.

The acetate buffer is used in the amount necessary to adjust pH of the composition to 3.8-4.2, preferably 4.0.

The compositions of the invention can be administered by nasal instillation or, preferably, as a nasal spray. For this purpose, suitable dosers are used delivering 0.1 ml of the composition for unit dose, which is equivalent to 25-250 I.U. of calcitonin, according to the above mentioned preferred calcitonin concentrations.

The following examples further illustrate the invention. All the described operation are effected under nitrogen.

EXAMPLE A

1,000,000 I.U. of salmon calcitonin is dissolved in 800 ml of bidistilled water, and a standard acetate buffer solution is added thereto to pH about 4, then 0.12 g of cetylpyridinium chloride and again bidistilled water to 1,000 ml final volume are added. The solution is filtered through a filter candle and filled into spray dosers delivering 0.1 ml per unit dose (= 100 I.U. of calcitonin).

EXAMPLE B

The procedure according to Example A is repeated so that the delivered unit dose of nasal composition (0.1 ml) contains 150 I.U of salmon calcitonin.

EXAMPLE C

The procedure of Example A is repeated, but with half the amount of calcitonin. Therefore, the nasal sprays will deliver 50 I.U. of salmon calcitonin per unit dose.

The compositions of the invention proved to be stable to contamination by such agents as :

| | | |
|---|---|---|
| Aspergillus niger | ATCC | 16404 |
| Candida albicans | ATCC | 10231 |
| Escherichia coli | ATCC | 8739 |
| Pseudomonas aeruginosa | ATCC | 9027 |
| Staphylococcus aureus | ATCC | 6538 |

which were added to the compositions in amounts of about $10^5$ micro-organisms in the inoculated liquid (according to the procedure described in Acta Pharm. Technol. 22, 247, (1976)), and after incubation for some hours, turned out to be nearly disappeared. No alive cells were revealed by a control effected on the solution two months later.

Moreover, the composition of Example A was compared with a composition of the same calcitonin concentration, prepared according to Italian Pat. N. 1,172,324. The test was carried out on 20 healthy volunteers of both sexes : after administration of doses of 100 I.U. calcitonin, blood withdrawals were effected at different times and calcitonin plasma levels (in pc/ml of plasma) were evaluated. The mean results can be summarized as follows :

| | It. Pat. | Ex. A |
|---|---|---|
| 5th min. | 24 | 28 |
| 10th min. | 53 | 61 |
| 20th min. | 73 | 82 |
| 40th min. | 18 | 24 |
| 60th min. | 5 | 10 |

The above data clearly show that the increase in bio-availability is markedly significant.

**Claims**

1. Intranasal aqueous pharmaceutical compositions containing calcitonin, with pH adjusted to 3.8-4.2 by means of acetate buffer, characterized in that they contain cetylpyridinium chloride.

2. Pharmaceutical compositions as claimed in claim 1, containing from about 100 to about 5,000 I.U. of calcitonin and from 0.01 to 0.4 mg of cetylpyridinium chloride per ml of solution.

3. Pharmaceutical compositions as claimed in the preceding claims, containing from about 25 to about 2,500 I.U. of calcitonin and from 0.05 to 0.2 mg of cetylpyridinium chloride per ml of solution.

4. Pharmaceutical compositions as claimed in the preceding claims, buffered to pH 4.

5. Pharmaceutical compositions as claimed in the preceding claims, in form of spray to be administered in form of unit doses equivalent to 25-250 I.U. of calcitonin.

European Patent Office

# EUROPEAN SEARCH REPORT

Application Number

EP 91 11 8477

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | WO-A-9 003 796 (SCHIAPPARELLI SALUTE)<br>* page 2, line 18 - line 26 *<br>* page 4, line 7 - line 9 *<br>--- | 1-5 | C07K15/00<br>A61K9/06 |
| Y | EP-A-0 115 627 (ARMOUR PHARMACEUTICAL COMPANY)<br>* page 5, line 19 - line 22 *<br>--- | 1-5 | |
| Y | EP-A-0 193 372 (TEIJIN LIMITED)<br>* column 2, line 2 *<br>* column 3, line 24 - line 41 *<br>--- | 1-5 | |
| Y<br><br>D | GB-A-2 127 689 (SANDOZ)<br>* the whole document *<br>& IT-A-1 172 324<br>--- | 1-5 | |
| Y | PATENT ABSTRACTS OF JAPAN<br>vol. 12, no. 279 (C-517)(3126) 1 August 1988<br>& JP-A-63 057 527 ( TOYO JOZO ) 12 March 1988<br>* abstract *<br>--- | 1-5 | |
| A | EP-A-0 093 373 (BERNSTEIN, J. ET AL)<br>* page 4, line 11; claim 12 *<br>----- | 1-5 | **TECHNICAL FIELDS SEARCHED (Int. Cl.5 )**<br>C07K<br>A61K |

The present search report has been drawn up for all claims

| Place of search | Date of completion of the search | Examiner |
|---|---|---|
| BERLIN | 30 JANUARY 1992 | AVEDIKIAN P.F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)